# FASCICULE DE BREVET EUROPEEN

(11) **EP 4 271 290 B1**
(45) Date de publication et mention de la délivrance du brevet: **02.10.2024**
(21) Numéro de dépôt: 21840062.0
(22) Date de dépôt: 23.12.2021
(51) Int. Cl.: A61B 17/66, A61B 17/68, A61B 17/00

(54) **OUTIL D'ACTIVATION POUR UN APPAREIL D'EXPANSION OSSEUSE**
AKTIVIERUNGSWERKZEUG FÜR EINE KNOCHENEXPANSIONSVORRICHTUNG
ACTIVATION TOOL FOR A BONE EXPANSION APPARATUS

(30) Priorité: 30.12.2020 FR 2014283
(43) Date de publication de la demande: 08.11.2023
(73) Titulaire: École Nationale Supérieure de Techniques Avancées, 91120 Palaiseau (FR); ASSISTANCE PUBLIQUE HOPITAUX DE PARIS, 75004 Paris (FR)
(72) Inventeur: KADLUB, Natacha, 75011 Paris (FR); BOISSON, Jean, 75011 Paris (FR); DALLARD, Jeremy, 94340 Joinville-Le-Pont (FR); SU, Emilie, 93000 Montreuil (FR)
(74) Mandataire: LLR
(86) Numéro de dépôt international: PCT/EP2021/087472
(87) Numéro de publication internationale: WO 2022/144296

(56) Documents cités:
- WO-A1-2008/003952
- WO-A1-2018/165243
- WO-A1-2020/069627
- CN-A- 111 920 502
- FR-A1- 2 906 453

## Description

L'invention concerne le domaine des appareils d'expansion osseuse, tels que, par exemple, un distracteur à plaques.

On connaît déjà dans l'état la technique, notamment d'après le document WO 2017/097998 A1, un distracteur à plaques prévu pour opérer une distraction maxillo-faciale. Un tel distracteur à plaques est fixé sur un côté d'un os ou sur le côté d'os voisins d'un sujet et comprend un aimant rotatif à proximité d'une extrémité du distracteur à plaques.

Une mise en rotation de cet aimant, à partir d'un outil d'activation externe également porteur d'un aimant, autour d'un axe de rotation coïncidant avec l'axe de rotation de l'aimant du distracteur, permet d'opérer une distraction osseuse. Pour ce faire, la rotation de l'aimant du distracteur entraîne l'écartement des plaques du distracteur par le biais d'un mécanisme de vis sans fin. La vis sans fin opère une conversion du mouvement de rotation de l'aimant du distracteur en un mouvement de translation d'une plaque du distracteur par rapport à l'autre plaque, ce qui entraîne un écartement des plaques.

L'ensemble de distraction osseuse décrit dans ce document comprend un outil d'activation externe au distracteur, dont la mise en rotation permet la rotation de l'aimant du distracteur à plaques par couplage magnétique. Il suffit dans ce cas de placer l'élément magnétique d'activation et l'élément magnétique distant porté par l'appareil d'expansion osseuse en vis-à-vis, ou en d'autres termes en regard l'un de l'autre. De cette manière, avec une telle configuration coaxiale, la rotation de l'élément magnétique d'activation sur lui-même autour d'un axe de rotation d'activation provoque la rotation de l'élément magnétique distant porté par l'appareil d'expansion osseuse sur lui-même autour d'un axe de distraction.

Dans cette configuration coaxiale, l'axe de rotation de l'élément magnétique distant, également appelé axe de distraction, coïncide avec l'axe de rotation de l'élément magnétique de l'outil d'activation. Ainsi, la rotation de l'élément magnétique distant porté par l'appareil d'expansion osseuse est obtenue via l'interaction coaxiale entre l'élément magnétique de l'outil d'activation et l'élément magnétique distant porté par l'appareil d'expansion osseuse, sans générer de force d'arrachement de l'appareil d'expansion osseuse selon une direction perpendiculaire à l'axe de distraction. Cela est particulièrement avantageux lorsque l'appareil d'expansion osseuse est un distracteur à plaques. En effet, un tel distracteur à plaques est souvent utilisé sur de très jeunes enfants et est par exemple fixé sur les os à l'aide de vis de courte longueur, par exemple de 5 mm de longueur, voire avec des vis résorbables en matière plastique. L'enlèvement du distracteur à plaques s'effectue par arrachement. Ainsi, la fixation du distracteur à plaques sur les os est relativement fragile et le réglage de l'écartement entre les plaques de fixation du distracteur à plaques ne doit pas provoquer de contraintes mécaniques susceptibles de provoquer un arrachement prématuré. C'est pourquoi l'intensité des contraintes mécaniques susceptibles de s'exercer sur la fixation des plaques aux os doit rester la plus faible possible pour permettre l'actionnement du distracteur à plaques en toute sécurité.

Du fait de ces faibles contraintes mécaniques, un blocage en rotation de l'élément magnétique distant peut survenir. Toutefois, cet outil d'activation est utilisé manuellement et ne permet pas de détecter facilement une défaillance au niveau du distracteur à plaques.

On connaît également d'après le document WO 2008/003952 A1, un dispositif distracteur, de préférence à plaques, comportant un élément magnétique pouvant être mis en rotation au moyen d'un actionneur magnétique externe.

L'invention a notamment pour but de faciliter la détection d'une défaillance du fonctionnement d'un appareil d'expansion osseuse, de préférence d'un distracteur à plaques.

A cet effet, l'invention a notamment pour objet un outil d'activation pour un appareil d'expansion osseuse, de préférence pour un distracteur à plaques, caractérisé en ce que l'outil d'activation comprend :
- un organe d'entraînement doté d'une sortie d'entraînement,
- un premier arbre configuré pour être entraîné en rotation autour d'un axe de rotation par la sortie d'entraînement,
- un élément magnétique d'activation, l'élément magnétique d'activation étant configuré pour interagir, de préférence coaxialement, avec un élément magnétique distant porté par l'appareil d'expansion osseuse,
- un deuxième arbre, disposé coaxialement au premier arbre, le deuxième arbre supportant l'élément magnétique d'activation,
- un dispositif d'accouplement interposé entre le premier arbre et le deuxième arbre, lequel est configuré pour coupler le premier arbre et le deuxième arbre dans un premier mode dit d'activation lorsque le premier arbre et le deuxième arbre possèdent la même vitesse de rotation, et est configuré pour découpler angulairement le premier arbre et le deuxième arbre dans un deuxième mode dit de défaillance lorsqu'une vitesse de rotation du deuxième arbre est plus élevée que la vitesse de rotation du premier arbre,
- des moyens de détection du basculement du premier mode dans le deuxième mode.

Grâce au dispositif d'accouplement prévu dans l'outil d'activation, on détecte aisément un blocage en rotation de l'élément magnétique distant, par exemple sans nécessiter de capteur de champ magnétique détectant la position angulaire de l'élément magnétique distant. Ainsi, la détection du basculement du premier mode dans le deuxième mode, ce qui correspond au découplage angulaire du premier arbre et du deuxième arbre, permet facilement de détecter une défaillance de la rotation de l'élément magnétique distant. Par conséquent, une défaillance de l'activation est aisément détectable. Par exemple, on peut ainsi se passer d'une vérification de la rotation de l'élément magnétique distant par des capteurs externes nécessitant d'être positionnés correctement vis-à-vis de l'élément magnétique distant.

Suivant d'autres caractéristiques optionnelles de l'outil d'activation, prises seules ou en combinaison :
- L'organe d'entraînement comprend un moteur électrique.
- L'élément magnétique d'activation est un aimant permanent aimanté selon une direction perpendiculaire à l'axe de rotation, de préférence à symétrie axiale par rapport à l'axe de rotation.
- Le deuxième arbre supporte axialement l'élément magnétique d'activation. Ainsi, la rotation du deuxième arbre entraîne une rotation de l'élément magnétique sur lui-même autour de l'axe de rotation, l'interaction coaxiale avec l'élément magnétique distant étant alors optimale.
- Le dispositif d'accouplement comporte un premier organe d'accouplement porté par le premier arbre, et un deuxième organe d'accouplement porté par le deuxième arbre, l'un parmi le premier organe d'accouplement et le deuxième organe d'accouplement comportant une première butée angulaire et une deuxième butée angulaire, la première butée angulaire et la deuxième butée angulaire étant décalées angulairement et délimitant entre elles un logement angulaire s'étendant sur un angle d'au moins 90° autour de l'axe de rotation, l'autre parmi le premier organe d'accouplement et le deuxième organe d'accouplement comportant un élément en saillie, l'élément en saillie étant engagé dans le logement angulaire de telle sorte que le deuxième arbre est librement pivotant autour de l'axe de rotation entre une première position angulaire dans laquelle l'élément en saillie se trouve en contact avec la première butée angulaire et une deuxième position angulaire dans laquelle l'élément en saillie se trouve en contact avec la deuxième butée angulaire. Ainsi, le dispositif d'accouplement est réalisé de manière particulièrement simple.
- L'angle entre la première position angulaire et la deuxième position angulaire est d'au moins 65°, de préférence d'au moins 90°, plus préférentiellement d'au moins 180°.
- Les moyens de détection détectent le pivotement du deuxième arbre hors de la première position angulaire lorsque le premier arbre est entraîné en rotation dans un premier sens de rotation, et le pivotement du deuxième arbre hors de la deuxième position angulaire lorsque le premier arbre est entraîné en rotation dans un deuxième sens de rotation opposé au premier sens de rotation.
- L'élément en saillie est un pion ou un secteur angulaire sous la forme d'une portion de disque.
- La première butée angulaire et la deuxième butée angulaire forment les extrémités angulaires d'un secteur angulaire sous la forme d'une portion de disque.
- Le logement angulaire s'étend sur un angle compris entre 90° et 300° autour de l'axe de rotation, préférentiellement égal à 295°. Ainsi, l'étendue angulaire du logement angulaire est suffisamment importante pour permettre une détection aisée du basculement du premier mode au deuxième mode, et donc pour permettre une détection d'un blocage de l'élément magnétique distant.
- Le logement angulaire s'étend sur un angle compris entre 90° et 170° autour de l'axe de rotation. Ainsi, les oscillations de l'élément magnétique d'activation sont diminuées lorsque celui-ci est découplé angulairement de la sortie d'entraînement du fait d'une défaillance de la rotation de l'élément magnétique distant. En effet, lors du basculement du premier mode dans le deuxième mode, le deuxième arbre est angulairement découplé du premier arbre. L'élément en saillie et l'une des deux butées angulaires perdent alors le contact entre eux, puis l'élément en saillie et l'autre des deux butées angulaires se contactent angulairement, l'inertie du premier arbre exerçant alors une fonction d'amortissement.
- Les moyens de détection comportent un indicateur visuel de la position angulaire du premier arbre et un indicateur visuel de la position angulaire du deuxième arbre. Ainsi, l'utilisateur peut aisément détecter le basculement du premier mode dans lequel les indicateurs visuels sont immobiles l'un par rapport à l'autre dans le deuxième mode dans lequel les indicateurs visuels se déplacent l'un par rapport à l'autre.
- Les moyens de détection comportent un premier capteur de la position angulaire du premier arbre et un deuxième capteur de la position angulaire du deuxième arbre. De préférence, les moyens de détection comprennent une mémoire de stockage configurée pour stocker les données de position angulaires du premier arbre et du deuxième arbre. Ainsi, un praticien peut prendre connaissance de ces données a posteriori, par exemple de manière hebdomadaire.
- Les moyens de détection comportent un capteur de couple configuré pour mesurer le couple exercé par le moteur électrique et/ou un capteur de courant configuré pour mesurer le courant consommé par le moteur électrique. Ainsi, on dispose d'un moyen très simple pour permettre une détection aisée du basculement du premier mode au deuxième mode, et donc pour permettre une détection d'un blocage de l'élément magnétique distant.
- Les moyens de détection comportent un indicateur visuel du basculement du premier mode au deuxième mode. De préférence, l'indicateur visuel du basculement du premier mode au deuxième mode comprend une diode électroluminescente verte qui est allumée lorsque l'entraînement est réalisé uniquement selon le premier mode, et comprend une diode électroluminescente rouge qui est allumée lorsque l'entraînement est réalisé avec au moins un basculement du premier mode au deuxième mode. Ainsi, une fois l'entraînement achevé, l'utilisateur est informé d'un fonctionnement correct via la diode électroluminescente verte, et est informé d'un fonctionnement défaillant via la diode électroluminescente rouge.
- Les moyens de détection comportent un indicateur sonore, lequel émet un signal sonore lorsque l'entraînement est réalisé uniquement selon le premier mode. Ainsi, une fois l'entraînement achevé, l'utilisateur est informé d'un fonctionnement correct via ce signal sonore.
- La vitesse de rotation de l'élément magnétique d'activation dans le premier mode est inférieure ou égale à 30 tours par minute, de préférence inférieure ou égale à 18 tours par minute. Ainsi, la détection par l'utilisateur d'un basculement de l'outil d'activation du premier mode dit d'activation dans le deuxième mode dit de défaillance est optimale, du fait que ce basculement s'effectue sur une durée suffisante pour que l'utilisateur puisse détecter visuellement une variation soudaine de la vitesse de rotation de l'élément magnétique d'activation et/ou une vibration et/ou une variation sonore du fait que le moteur électrique ne transmet plus de couple au deuxième arbre dans le deuxième mode dit de défaillance. Plus précisément, la vitesse de rotation est suffisamment faible pour que, lorsque l'élément magnétique distant ne tourne plus en interaction avec l'élément magnétique d'activation, le couple exercé par l'élément magnétique distant sur l'élément magnétique d'activation génère une augmentation de la vitesse de rotation de l'élément magnétique d'activation, et par conséquent une augmentation de la vitesse de rotation du deuxième arbre. Ainsi, cela génère une perte de contact entre l'élément en saillie et l'une parmi la première butée angulaire et la deuxième butée angulaire, selon le sens de rotation du moteur électrique.
- La vitesse de rotation de l'élément magnétique d'activation est constante dans le premier mode dit d'activation. Ainsi, une variation de vitesse de l'élément magnétique d'activation est facilement détectée lorsque l'outil d'activation bascule dans le deuxième mode dit de défaillance.
- L'organe d'entraînement, de préférence le moteur électrique, est configuré de telle sorte que la sortie d'entraînement est entraînée tour par tour. En effet, l'activation du distracteur à plaques est délicate et doit être opérée par étapes afin d'éviter une sollicitation trop importante du distracteur à plaques. Par étape, il faut par exemple comprendre que l'organe d'entraînement entraîne en rotation sur un tour la sortie d'entraînement et le premier arbre, puis est arrêté, la rotation de la sortie d'entraînement et du premier arbre étant alors stoppée. Par exemple, l'étape précitée est réitérée chaque jour.
- L'outil d'activation comporte un roulement à billes disposé sur le deuxième arbre. Ainsi, la présence d'un roulement à billes permet, grâce aux forces de frottement générées par celui-ci, de diminuer les éventuelles oscillations de l'élément magnétique d'activation lorsque l'outil d'activation se trouve dans le deuxième mode dit de défaillance.
- L'outil d'activation comporte un boîtier et le roulement à billes est interposé entre le boîtier et le deuxième arbre.

L'invention a également pour objet un ensemble de distraction comportant :
- un outil d'activation du type précédemment décrit, dans lequel l'élément magnétique d'activation est un aimant permanent aimanté selon une direction perpendiculaire à l'axe de rotation, et
- un distracteur à plaques, lequel comporte une première plaque de fixation et une deuxième plaque de fixation, un organe de distraction configuré pour modifier la distance entre la première plaque de fixation et la deuxième plaque de fixation, et un élément magnétique distant sous forme d'un aimant permanent, configuré pour entraîner l'organe de distraction par rotation sur lui-même autour d'un axe de distraction, l'élément magnétique distant étant un aimant permanent aimanté selon une direction perpendiculaire à l'axe de distraction.

Suivant d'autres caractéristiques optionnelles de l'ensemble de distraction, prises seules ou en combinaison :
- L'ensemble de distraction est tel que, lorsque l'axe de distraction coïncide avec l'axe de rotation : dans le premier mode, l'élément magnétique distant est entraîné en rotation par l'élément magnétique d'activation ; dans le deuxième mode, l'élément magnétique distant entraîne en rotation l'élément magnétique d'activation jusqu'à ce que leurs directions d'aimantation soient parallèles.
- L'organe de distraction comprend une tige filetée coopérant avec un trou fileté dans une plaque de fixation telle que la première plaque de fixation, de telle sorte que le pas du filetage de la tige filetée est compris entre 0,20 et 0,25 mm par tour, de préférence égal à 0,20 mm par tour. Ainsi, lors de la rotation sur un tour de la tige filetée, la distance entre les plaques de fixation est modifiée de la valeur correspondant au pas du filetage, les plaques de fixation se rapprochant ou s'éloignant selon le sens de rotation.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue schématique en coupe de face d'un ensemble de distraction comportant un outil d'activation selon une première variante de réalisation ;
[Fig. 2] la figure 2 est une vue schématique en perspective de l'outil d'activation selon la première variante de réalisation ;
[Fig. 3] la figure 3 est un ensemble de vues schématiques en coupe transversale selon le plan A-A de l'outil d'activation selon la première variante de réalisation dans un premier mode dit d'activation.
[Fig. 4] la figure 4 est un ensemble de vues schématiques en coupe transversale selon le plan A-A de l'outil d'activation selon la première variante de réalisation dans un deuxième mode dit de défaillance.
[Fig. 5] la figure 5 est une vue schématique en coupe de face d'un ensemble de distraction comportant un outil d'activation selon une deuxième variante de réalisation.
[Fig. 6] la figure 6 est un ensemble de vues schématiques en coupe transversale selon le plan B-B de l'outil d'activation selon la deuxième variante de réalisation dans un deuxième mode dit de défaillance.

### Description détaillée

Sur toutes les figures, les mêmes références se rapportent aux mêmes éléments. Dans cette description détaillée, les réalisations suivantes sont des exemples. Bien que la description se réfère à un ou plusieurs modes de réalisation, cela ne signifie pas que les caractéristiques s'appliquent seulement à une seule variante de réalisation. De simples caractéristiques de différentes variantes de réalisation peuvent également être combinées et/ou interchangées pour fournir d'autres réalisations.

La présente description désigne certains éléments ou organes par des noms ou expressions, comme par exemple « premier arbre » ou « deuxième arbre ». Dans ce cas, il s'agit de désignations pour différencier et dénommer des éléments ou organes non identiques. Ces désignations n'impliquent pas une priorité ou une hiérarchie d'un élément (ou d'un organe) par rapport à un autre et il est aisément possible d'i nterchanger de telles dénominations sans sortir du cadre de la présente description. Ces désignations n'impliquent pas non plus un ordre dans le temps par exemple pour apprécier tels ou tels critères.

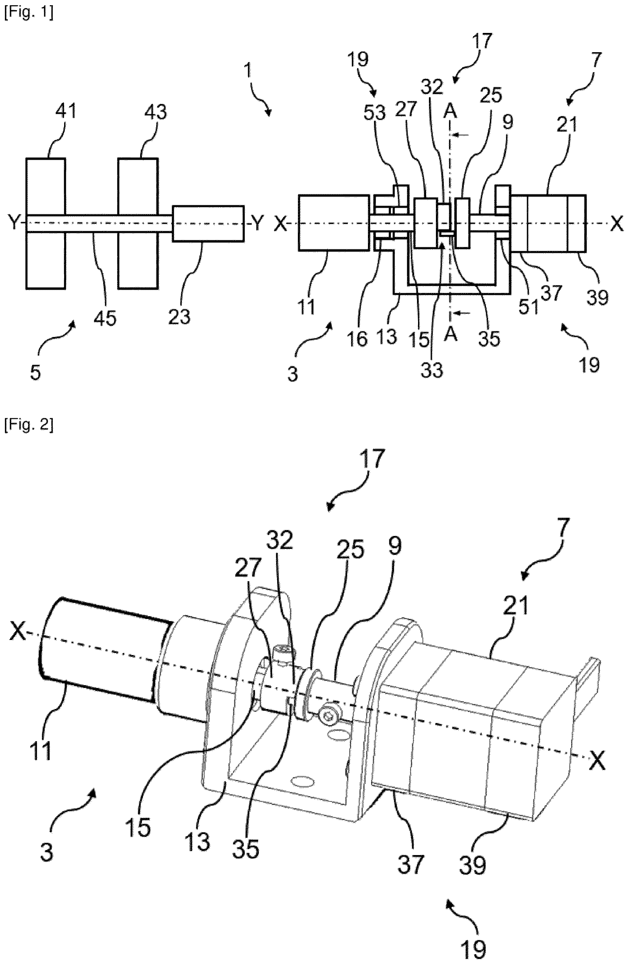

On a représenté schématiquement sur la figure 1 un ensemble de distraction selon une première variante de réalisation, désigné par la référence générale 1.

L'ensemble de distraction 1 comprend un outil d'activation 3 et un distracteur à plaques 5.

L'outil d'activation 3 comprend : un organe d'entraînement 7 doté d'une sortie d'entraînement, un premier arbre 9 configuré pour être entraîné en rotation autour d'un axe de rotation XX par la sortie d'entraînement, et un élément magnétique d'activation 11.

L'outil d'activation 3 comprend également un deuxième arbre 15, disposé coaxialement au premier arbre 9, le deuxième arbre 15 supportant l'élément magnétique d'activation 11. Un roulement à billes 16 est disposé sur le deuxième arbre 15. Plus précisément, l'outil d'activation 3 comporte un boîtier 13 et le roulement à billes 16 est interposé entre le boîtier 13 et le deuxième arbre 15. Par ailleurs, le boîtier 13 supporte l'organe d'entraînement 7.

L'outil d'activation 3 comprend en outre un dispositif d'accouplement 17 interposé entre le premier arbre 9 et le deuxième arbre 15, lequel est configuré pour coupler le premier arbre 9 et le deuxième arbre 15 dans un premier mode dit d'activation lorsque le premier arbre 9 et le deuxième arbre 15 possèdent la même vitesse de rotation, et est configuré pour découpler angulairement le premier arbre 9 et le deuxième arbre 15 dans un deuxième mode dit de défaillance lorsqu'une vitesse de rotation du deuxième arbre 15 est plus élevée que la vitesse de rotation du premier arbre 9. Enfin, l'outil d'activation 3 comprend des moyens de détection 19 du basculement du premier mode dans le deuxième mode.

Dans cet exemple, l'organe d'entraînement 7 comprend un moteur électrique 21, lequel entraîne en rotation le premier arbre 9 via la sortie d'entraînement.

L'organe d'entraînement 7, de préférence le moteur électrique 21, est configuré de telle sorte que la sortie d'entraînement est entraînée tour par tour. L'organe d'entraînement 7 entraîne en rotation sur un tour la sortie d'entraînement et le premier arbre 9, puis est arrêté, la rotation de la sortie d'entraînement et du premier arbre 9 étant alors stoppée. Par exemple, l'étape précitée est réitérée chaque jour.

L'élément magnétique d'activation 11 est supporté axialement par le deuxième arbre 15. L'élément magnétique d'activation 11 est configuré pour interagir, de préférence coaxialement, avec un élément magnétique distant 23 porté par le distracteur à plaques 5. Dans cet exemple, l'élément magnétique d'activation 11 est un aimant permanent aimanté selon une direction Da perpendiculaire à l'axe de rotation XX, de préférence à symétrie axiale par rapport à l'axe de rotation XX. La vitesse de rotation de l'élément magnétique d'activation 11 dans le premier mode est inférieure ou égale à 30 tours par minute, de préférence inférieure ou égale à 18 tours par minute. Par exemple, la vitesse de rotation de l'élément magnétique d'activation 11 est constante dans le premier mode.

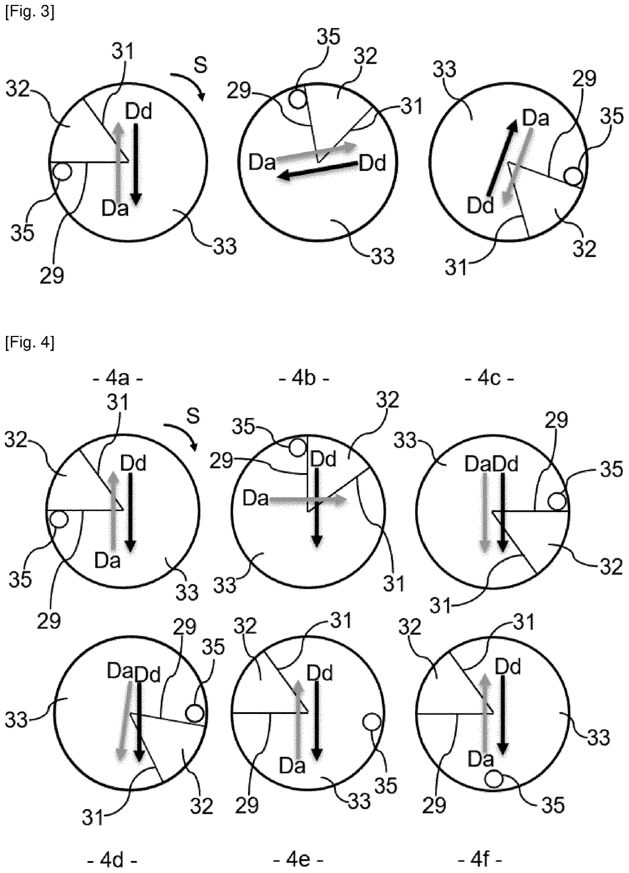

Le dispositif d'accouplement 17 comporte un premier organe d'accouplement 25 porté par le premier arbre 9, et un deuxième organe d'accouplement 27 porté par le deuxième arbre 15. Dans cet exemple, notamment visible sur les figures 3 et 4, le deuxième organe d'accouplement 27 comporte une première butée angulaire 29 et une deuxième butée angulaire 31, la première butée angulaire 29 et la deuxième butée angulaire 31 étant décalées angulairement et délimitant entre elles un logement angulaire 33 s'étendant sur un angle d'au moins 90° autour de l'axe de rotation XX. La première butée angulaire 29 et la deuxième butée angulaire 31 forment les extrémités angulaires d'un secteur angulaire 32 sous la forme d'une portion de disque. Le premier organe d'accouplement 25 comporte un élément en saillie 35, dans cet exemple sous forme de pion. Selon une variante non représentée, l'élément en saillie 35 est un secteur angulaire sous la forme d'une portion de disque. L'élément en saillie 35 est engagé dans le logement angulaire 33 de telle sorte que le deuxième arbre 15 est librement pivotant autour de l'axe de rotation XX entre une première position angulaire dans laquelle l'élément en saillie 35 se trouve en contact avec la première butée angulaire 29 et une deuxième position angulaire dans laquelle l'élément en saillie 35 se trouve en contact avec la deuxième butée angulaire 31.

Le logement angulaire 33 s'étend sur un angle compris entre 90° et 300° autour de l'axe de rotation, préférentiellement égal à 295° comme représenté dans cet exemple.

Par ailleurs, l'angle entre la première position angulaire et la deuxième position angulaire est d'au moins 65°, de préférence d'au moins 90°, plus préférentiellement d'au moins 180°, encore plus préférentiellement d'au moins 270°. Dans cet exemple, cet angle est de 275°.

Les moyens de détection 19 détectent le pivotement du deuxième arbre 15 hors de la première position angulaire lorsque le premier arbre 9 est entraîné en rotation dans un premier sens de rotation S, et le pivotement du deuxième arbre 15 hors de la deuxième position angulaire lorsque le premier arbre 9 est entraîné en rotation dans un deuxième sens de rotation opposé au premier sens de rotation S.

Les moyens de détection 19 comportent un capteur de couple 37 configuré pour mesurer le couple exercé par le moteur électrique 21 et/ou un capteur de courant 39 configuré pour mesurer le courant consommé par le moteur électrique 21. Alternativement ou de manière complémentaire, les moyens de détection 19 comportent un premier capteur 51 de la position angulaire du premier arbre 9 et un deuxième capteur 53 de la position angulaire du deuxième arbre 15, lesquels sont par exemple intégrés dans le boîtier 13. De préférence, les moyens de détection 19 comprennent une mémoire de stockage configurée pour stocker les données de position angulaires du premier arbre 9 et du deuxième arbre 15.

Les moyens de détection 19 comportent un indicateur visuel du basculement du premier mode au deuxième mode. De préférence, l'indicateur visuel du basculement du premier mode au deuxième mode comprend une diode électroluminescente verte qui est allumée lorsque l'entraînement est réalisé selon le premier mode uniquement, et comprend une diode électroluminescente rouge qui est allumée lorsque l'entraînement est réalisé avec au moins un basculement du premier mode au deuxième mode.

Les moyens de détection 19 comportent un indicateur sonore, lequel émet un signal sonore lorsque l'entraînement est réalisé uniquement selon le premier mode.

Si le moteur électrique 21 fait un tour complet sans qu'il y ait découplage : l'activation est prise en compte, la diode électroluminescente verte s'allume et le signal sonore retentit. Si le moteur électrique 21 fait un tour complet et qu'il y a eu découplage, plus précisément un basculement du premier mode dans le deuxième mode : la diode électroluminescente rouge s'allume. La position angulaire à laquelle se trouve l'élément magnétique distant 23 est par exemple déterminée de la manière suivante. A partir de la position angulaire du deuxième arbre 15, de la position angulaire du premier arbre 9 et du couple du moteur électrique 21, on détermine la position angulaire du premier arbre 9 entraîné par le moteur électrique 21 au moment du basculement dans le deuxième mode, et il faut retirer de l'ordre de 180 degrés, par exemple 181 degrés, à cet angle pour retrouver la valeur de la position angulaire de l'élément magnétique distant 23.

Le distracteur à plaques 5 comporte une première plaque de fixation 41 et une deuxième plaque de fixation 43, un organe de distraction 45 configuré pour modifier la distance entre la première plaque de fixation 41 et la deuxième plaque de fixation 43, et l'élément magnétique distant 23 sous forme d'un aimant permanent.

L'organe de distraction 45 comprend une tige filetée coopérant avec un trou fileté dans une plaque de fixation 41, 43 telle que la première plaque de fixation 41, de telle sorte que le pas du filetage de la tige filetée est compris entre 0,20 et 0,25 mm par tour, de préférence égal à 0,20 mm par tour.

L'élément magnétique distant 23 est configuré pour entraîner l'organe de distraction 45 par rotation sur lui-même autour d'un axe de distraction YY, l'élément magnétique distant 23 étant un aimant permanent aimanté selon une direction Dd perpendiculaire à l'axe de distraction YY.

L'ensemble de distraction 1 est tel que, lorsque l'axe de distraction YY coïncide avec l'axe de rotation XX : dans le premier mode, l'élément magnétique distant 23 est entraîné en rotation par l'élément magnétique d'activation 11, comme représenté sur la figure 3 ; dans le deuxième mode, l'élément magnétique distant 23 entraîne en rotation l'élément magnétique d'activation 11 jusqu'à ce que leurs directions d'aimantation Da, Dd soient parallèles, plus précisément parallèles et opposées.

Dans le premier mode représenté sur la figure 3, l'élément magnétique distant 23 est entraîné en rotation selon le premier sens de rotation S par l'élément magnétique d'activation 11 par effet magnétique, du fait que leurs directions d'aimantation Da, Dd sont parallèles, plus précisément parallèles et opposées. Lorsque l'élément magnétique d'activation 11 pivote, l'élément magnétique distant 23 pivote afin de maintenir leurs directions d'aimantation Da, Dd parallèles, plus précisément parallèles et opposées.

Le basculement dans le deuxième mode d'activation a lieu lorsque leurs directions d'aimantation Da, Dd deviennent non parallèles comme représenté à l'étape 4d sur la figure 4, par exemple du fait du blocage en rotation de l'élément magnétique distant 23 alors que l'élément magnétique d'activation 11 est entraîné en rotation.

La figure 4 représente via ses vues 4a à 4f des étapes du fonctionnement de l'ensemble de distraction 1 lorsque l'élément magnétique distant 23 se trouve bloqué en rotation, le premier sens de rotation S du premier arbre 9 et de l'élément en saillie 35 étant indiqué sur la vue 4a. Dans ce cas, lorsque l'élément magnétique d'activation 11 est entraîné en rotation, l'élément magnétique d'activation 11 n'entraîne plus en rotation l'élément magnétique distant 23.

Sur la vue 4a, l'élément en saillie 35 est en contact avec la première butée angulaire 29. A ce stade, les directions d'aimantation Da et Dd sont parallèles et opposées.

Sur la vue 4b, l'élément en saillie 35 est encore en contact avec la première butée angulaire 29. A ce stade, les directions d'aimantation Da et Dd ne sont plus parallèles. Néanmoins, l'élément en saillie 35 entraîne encore en rotation le deuxième organe d'accouplement 27 via la première butée angulaire 29, ce qui empêche le deuxième organe d'accouplement 27, le deuxième arbre 15 et l'élément magnétique d'activation 11 de pivoter dans un deuxième sens de rotation opposé au premier sens de rotation S afin de prendre une position d'équilibre vis-à-vis de l'élément magnétique distant 23, dans laquelle leurs directions d'aimantation Da, Dd sont parallèles et opposées.

Sur la vue 4c, l'élément en saillie 35 est encore en contact avec la première butée angulaire 29. A ce stade, les directions d'aimantation Da et Dd sont parallèles et dans le même sens. Néanmoins à ce stade, l'élément en saillie 35 entraîne encore en rotation le deuxième organe d'accouplement 27 via la première butée angulaire 29, ce qui empêche le deuxième organe d'accouplement 27 et l'élément magnétique d'activation 11 de prendre une position d'équilibre vis-à-vis de l'élément magnétique distant 23.

Sur la vue 4d, l'élément en saillie 35 quitte le contact avec la première butée angulaire 29. A ce stade, les directions d'aimantation Da et Dd ne sont plus parallèles, l'élément magnétique distant 23 entraîne en rotation l'élément magnétique d'activation 11. De ce fait, le deuxième organe d'accouplement 27, le deuxième arbre 15 et l'élément magnétique d'activation 11 pivotent librement selon le premier sens de rotation S afin de prendre une position angulaire d'équilibre vis-à-vis de l'élément magnétique distant 23, dans laquelle leurs directions d'aimantation Da, Dd sont parallèles et opposées, comme représenté sur la vue 4e.

Sur la vue 4f, le deuxième organe d'accouplement 27, le deuxième arbre 15 et l'élément magnétique d'activation 11 sont immobiles dans la position angulaire d'équilibre vis-à-vis de l'élément magnétique distant 23, mais l'élément en saillie 35 a continué à pivoter du fait de l'entraînement via le premier arbre 9 et le moteur électrique 21. Dans ce cas, du fait que le premier arbre 9 n'entraîne plus le deuxième arbre 15, le couple exercé par le moteur est nettement plus faible que lorsque le premier arbre entraîne le deuxième arbre 15. L'élément en saillie 35 tend à se rapprocher de la position angulaire occupée par le deuxième organe d'accouplement, et finit par revenir en contact avec la première butée angulaire 29, comme représenté sur la vue 4a.

Lorsque l'entraînement du premier arbre 9 est opéré dans un deuxième sens de rotation opposé au premier sens de rotation S, le fonctionnement est similaire à celui exposé ci-dessus à la différence près que la première butée angulaire 29 est remplacée par la deuxième butée angulaire 31.

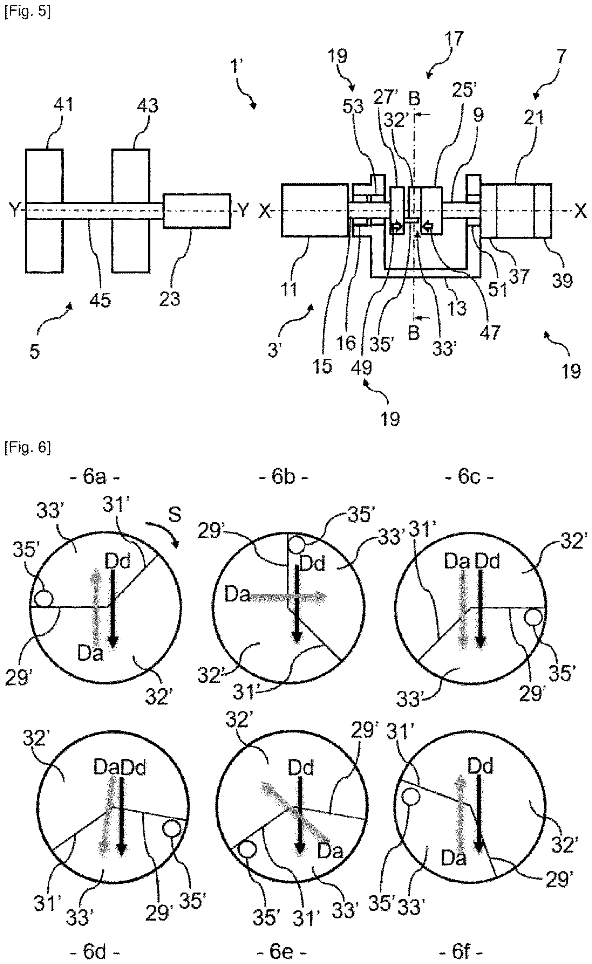

On a représenté schématiquement sur la figure 5 un ensemble de distraction selon une deuxième variante de réalisation, désigné par la référence générale 1'. Les éléments composant l'ensemble de distraction 1' selon la deuxième variante de réalisation sont similaires à ceux composant l'ensemble de distraction 1 selon la première variante de réalisation, à l'exception des éléments ci-après exposés.

Cet ensemble de distraction 1' selon la deuxième variante de réalisation se distingue de l'ensemble de distraction 1 selon la première variante de réalisation principalement en ce que dans l'outil d'activation 3', un élément en saillie 35', similaire à l'élément en saillie 35 de la première variante, est porté par le deuxième arbre 15, et en ce qu'un logement angulaire 33' est porté par le premier arbre 9.

Dans cet exemple, le premier organe d'accouplement 25' comporte une première butée angulaire 29' et une deuxième butée angulaire 31', la première butée angulaire 29' et la deuxième butée angulaire 31' étant décalées angulairement et délimitant entre elles le logement angulaire 33' s'étendant sur un angle compris entre 90° et 300°, de préférence entre 90° et 170°, et dans cet exemple égal à 135°. La première butée angulaire 29' et la deuxième butée angulaire 31' forment les extrémités angulaires d'un secteur angulaire 32' sous la forme d'une portion de disque. Le deuxième organe d'accouplement 27' comporte un élément en saillie 35'. L'élément en saillie 35' est engagé dans le logement angulaire 33' de telle sorte que l'élément en saillie 35' est librement pivotant autour de l'axe de rotation XX entre une première position angulaire dans laquelle l'élément en saillie 35' se trouve en contact avec la première butée angulaire 29' et une deuxième position angulaire dans laquelle l'élément en saillie 35' se trouve en contact avec la deuxième butée angulaire 31'.

Dans cette variante, les moyens de détection 19 comportent un premier indicateur visuel 47 de la position angulaire du premier arbre 9 et un deuxième indicateur visuel 49 de la position angulaire du deuxième arbre 15. Dans cet exemple, le premier indicateur visuel 47 est sous forme d'une flèche sur le premier organe d'accouplement 25', la flèche étant orientée vers le deuxième organe d'accouplement 27'. Le deuxième indicateur visuel 49 est sous forme d'une flèche sur le deuxième organe d'accouplement 27', la flèche étant orientée vers le premier organe d'accouplement 25'.

Le fonctionnement de l'ensemble de distraction 1' selon cette deuxième variante de réalisation est similaire au fonctionnement de l'ensemble de distraction 1 selon la première variante de réalisation.

La figure 6 représente via ses vues 6a à 6f des étapes du fonctionnement de l'ensemble de distraction 1' lorsque l'élément magnétique distant 23 se trouve bloqué en rotation, le premier sens de rotation S du premier arbre 9, du secteur angulaire 32' et des butées angulaires 29' et 31' étant indiqué sur la vue 6a. Dans ce cas, lorsque l'élément magnétique d'activation 11 est entraîné en rotation, l'élément magnétique d'activation 11 n'entraîne plus en rotation l'élément magnétique distant 23.

Toutefois, du fait que le logement angulaire 33' s'étend sur un angle compris entre 90° et 170° et dans cet exemple égal à 135°, lors du basculement du premier mode dans le deuxième mode, l'élément en saillie 35' est libéré du contact de la première butée angulaire 29' selon le premier sens de rotation S, comme représenté sur la vue 6d de la figure 6. A ce stade, les directions d'aimantation Da et Dd ne sont plus parallèles, l'élément magnétique distant 23 entraîne en rotation l'élément magnétique d'activation 11.

Comme représenté sur la vue 6e de la figure 6, l'élément en saillie 35' vient ensuite en contact angulairement avec la deuxième butée angulaire 31'. Par exemple, un tel contact est détectable par un utilisateur, soit du fait de la vibration due à cette mise en contact, soit du fait du son généré par cette mise en contact. Ainsi, dans cet exemple, les moyens de détection 19 comprennent également l'élément en saillie 35', la deuxième butée angulaire 31' lorsque le premier arbre 9 est entraîné en rotation dans le premier sens de rotation S, et la première butée angulaire 29' lorsque le premier arbre 9 est entraîné dans un deuxième sens de rotation opposé au premier sens de rotation S. Lors de cette mise en contact, la deuxième butée angulaire 31' exerce une fonction d'amortissement du fait de l'inertie notamment du premier arbre 9, jusqu'à ce que le deuxième organe d'accouplement 27', le deuxième arbre 15 et l'élément magnétique d'activation 11 prennent une position angulaire d'équilibre vis-à-vis de l'élément magnétique distant 23, dans laquelle leurs directions d'aimantation Da, Dd sont parallèles et opposées, comme représenté sur la vue 6f de la figure 6. A ce stade, les directions d'aimantation Da et Dd sont parallèles, plus précisément parallèles et opposées, et ni l'élément magnétique distant 23 ni la première butée angulaire 29' n'entraîne en rotation l'élément magnétique d'activation 11.

Lorsque l'entraînement du premier arbre 9 est opéré dans un deuxième sens de rotation opposé au premier sens de rotation S, le fonctionnement est similaire à celui exposé ci-dessus à la différence près que la première butée angulaire 29' est remplacée par la deuxième butée angulaire 31', et inversement.

L'invention n'est pas limitée aux modes de réalisation présentés et d'autres modes de réalisation apparaîtront clairement à l'homme du métier. Il est notamment possible de combiner les modes de réalisations entre eux lorsque cela est techniquement réalisable.

### Liste de références

1, 1' : ensemble de distraction
3, 3' : outil d'activation
5 : distracteur à plaques
7 : organe d'entraînement
9 : premier arbre
11 : élément magnétique d'activation
13 : boîtier
15 : deuxième arbre
16 : roulement à billes
17 : dispositif d'accouplement
19 : moyens de détection
21 : moteur électrique
23 : élément magnétique distant
25, 25' : premier organe d'accouplement
27, 27' : deuxième organe d'accouplement
29, 29' : première butée angulaire
31, 31' : deuxième butée angulaire
32, 32 : secteur angulaire
33, 33' : logement angulaire
35, 35' : élément en saillie
37 : capteur de couple
39 : capteur de courant
41 : première plaque de fixation
43 : deuxième plaque de fixation
45 : organe de distraction
47 : premier indicateur visuel
49 : deuxième indicateur visuel
51 : premier capteur
53 : deuxième capteur
XX : axe de rotation
YY : axe de distraction
Da : direction d'aimantation de l'élément magnétique d'activation
Dd : direction d'aimantation de l'élément magnétique distant
S : premier sens de rotation

## Revendications

1. Outil d'activation (3, 3') pour un appareil d'expansion osseuse, de préférence pour un distracteur à plaques (5), l'outil d'activation (3, 3') comprenant :
- un organe d'entraînement (7) doté d'une sortie d'entraînement,
- un premier arbre (9) configuré pour être entraîné en rotation autour d'un axe de rotation par la sortie d'entraînement,
- un élément magnétique d'activation (11) configuré pour interagir, de préférence coaxialement, avec un élément magnétique distant (23) porté par l'appareil d'expansion osseuse,
- un deuxième arbre (15), disposé coaxialement au premier arbre (9), le deuxième arbre (15) supportant l'élément magnétique d'activation (11),
**caractérisé en ce que** l'outil d'activation (3, 3') comprend :
- un dispositif d'accouplement (17) interposé entre le premier arbre (9) et le deuxième arbre (15), lequel est configuré pour coupler le premier arbre (9) et le deuxième arbre (15) dans un premier mode dit d'activation lorsque le premier arbre (9) et le deuxième arbre (15) possèdent la même vitesse de rotation, et est configuré pour découpler angulairement le premier arbre (9) et le deuxième arbre (15) dans un deuxième mode dit de défaillance lorsqu'une vitesse de rotation du deuxième arbre (15) est plus élevée que la vitesse de rotation du premier arbre (9), et
- des moyens de détection (19) du basculement du premier mode dans le deuxième mode,
le dispositif d'accouplement (17) comportant :
- un premier organe d'accouplement (25, 25') porté par le premier arbre (9), et
- un deuxième organe d'accouplement (27, 27') porté par le deuxième arbre (15),
l'un parmi le premier organe d'accouplement (25, 25') et le deuxième organe d'accouplement (27, 27') comportant une première butée angulaire (29, 29') et une deuxième butée angulaire (31, 31'), la première butée angulaire (29, 29') et la deuxième butée angulaire (31, 31') étant décalées angulairement et délimitant entre elles un logement angulaire (33, 33') s'étendant sur un angle d'au moins 90° autour de l'axe de rotation,
l'autre parmi le premier organe d'accouplement (25, 25') et le deuxième organe d'accouplement (27, 27') comportant un élément en saillie (35, 35'),
l'élément en saillie (35, 35') étant engagé dans le logement angulaire (33, 33') de telle sorte que le deuxième arbre (15) est librement pivotant autour de l'axe de rotation (XX) entre une première position angulaire dans laquelle l'élément en saillie (35, 35') se trouve en contact avec la première butée angulaire (29, 29') et une deuxième position angulaire dans laquelle l'élément en saillie (35, 35') se trouve en contact avec la deuxième butée angulaire (31, 31').

2. Outil d'activation (3, 3') selon la revendication précédente, dans lequel l'élément en saillie (35, 35') est un pion ou un secteur angulaire sous la forme d'une portion de disque, et dans lequel la première butée angulaire (29, 29') et la deuxième butée angulaire (31, 31') forment les extrémités angulaires d'un secteur angulaire sous la forme d'une portion de disque.

3. Outil d'activation (3, 3') selon la revendication précédente, dans lequel le logement angulaire (33, 33') s'étend sur un angle compris entre 90° et 300° autour de l'axe de rotation (XX).

4. Outil d'activation (3, 3') selon l'une quelconque des revendications précédentes, dans lequel l'organe d'entraînement (7) comprend un moteur électrique (21).

5. Outil d'activation (3, 3') selon l'une quelconque des revendications précédentes, dans lequel les moyens de détection (19) comportent un premier capteur (51) de la position angulaire du premier arbre (9) et un deuxième capteur (53) de la position angulaire du deuxième arbre (15).

6. Outil d'activation (3, 3') selon l'une quelconque des revendications précédentes, dans lequel les moyens de détection (19) comportent un capteur de couple (37) configuré pour mesurer le couple exercé par le moteur électrique (21) et/ou un capteur de courant (39) configuré pour mesurer le courant consommé par le moteur électrique (21).

7. Outil d'activation (3, 3') selon l'une quelconque des revendications précédentes, dans lequel la vitesse de rotation de l'élément magnétique d'activation (11) dans le premier mode est inférieure ou égale à 30 tours par minute, de préférence inférieure ou égale à 18 tours par minute.

8. Outil d'activation (3, 3') selon l'une quelconque des revendications précédentes, lequel comporte un roulement à billes (16) disposé sur le deuxième arbre (15).

9. Ensemble de distraction (1, 1') comportant :
- un outil d'activation (3, 3') selon l'une quelconque des revendications précédentes, dans lequel l'élément magnétique d'activation (11) est un aimant permanent aimanté selon une direction (Da) perpendiculaire à l'axe de rotation (XX), et
- un distracteur à plaques (5), lequel comporte une première plaque de fixation (41), une deuxième plaque de fixation (43), un organe de distraction (45) configuré pour modifier la distance entre la première plaque de fixation (41) et la deuxième plaque de fixation (43), et un élément magnétique distant (23) sous forme d'un aimant permanent, configuré pour entraîner l'organe de distraction (45) par rotation sur lui-même autour d'un axe de distraction (YY), l'élément magnétique distant (23) étant un aimant permanent aimanté selon une direction (Dd) perpendiculaire à l'axe de distraction (YY).

## Patentansprüche

1. Aktivierungswerkzeug (3, 3') für eine Knochenexpansionsvorrichtung, vorzugsweise für einen Plattendistraktor (5), wobei das Aktivierungswerkzeug (3, 3') Folgendes aufweist:
- ein Antriebselement (7), das mit einem Antriebsausgang versehen ist,
- eine erste Welle (9), die so konfiguriert ist, dass sie durch den Antriebsausgang um eine Drehachse gedreht wird,
- ein aktivierendes Magnetelement (11), das so konfiguriert ist, dass es mit einem entfernten Magnetelement (23), das von der Knochenexpansionsvorrichtung getragen wird, interagiert, vorzugsweise koaxial,
- eine zweite Welle (15), die koaxial zur ersten Welle (9) angeordnet ist, wobei die zweite Welle (15) das aktivierende Magnetelement (11) trägt,
**dadurch gekennzeichnet, dass** das Aktivierungswerkzeug (3, 3') aufweist:
- eine zwischen der ersten Welle (9) und der zweiten Welle (15) angeordnete Kupplungsvorrichtung (17), die so konfiguriert ist, dass sie die erste Welle (9) und die zweite Welle (15) in einem ersten sogenannten Aktivierungsmodus koppelt, wenn die erste Welle (9) und die zweite Welle (15) die gleiche Drehzahl haben, und so konfiguriert ist, dass sie die erste Welle (9) und die zweite Welle (15) in einem zweiten sogenannten Ausfallmodus winkelmäßig entkoppelt, wenn eine Drehgeschwindigkeit der zweiten Welle (15) höher ist als die Drehgeschwindigkeit der ersten Welle (9), und
- Mittel (19) zum Erfassen des Umschaltens vom ersten Modus in den zweiten Modus,
wobei die Kupplungsvorrichtung (17) aufweist:
- ein erstes Kupplungselement (25, 25'), das von der ersten Welle (9) getragen wird, und
- ein zweites Kupplungselement (27, 27'), das von der zweiten Welle (15) getragen wird,
wobei eines von dem ersten Kupplungselement (25, 25') und dem zweiten Kupplungselement (27, 27') einen ersten Winkelanschlag (29, 29') und einen zweiten Winkelanschlag (31, 31') aufweist, wobei der erste Winkelanschlag (29, 29') und der zweite Winkelanschlag (31, 31') winkelmäßig versetzt sind und zwischen sich eine Winkelaufnahme (33, 33') begrenzen, die sich über einen Winkel von mindestens 90° um die Drehachse erstreckt,
wobei das andere von dem ersten Kupplungselement (25, 25') und dem zweiten Kupplungselement (27, 27') ein vorstehendes Element (35, 35') aufweist,
wobei das vorstehende Element (35, 35') in die Winkelaufnahme (33, 33') eingreift, so dass die zweite Welle (15) frei um die Drehachse (XX) zwischen einer ersten Winkelposition, in der das vorstehende Element (35, 35') in Kontakt mit dem ersten Winkelanschlag (29, 29') steht, und einer zweiten Winkelposition, in der das vorstehende Element (35, 35') in Kontakt mit dem zweiten Winkelanschlag (31, 31') steht, schwenkbar ist.

2. Aktivierungswerkzeug (3, 3') nach dem vorhergehenden Anspruch, wobei das vorstehende Element (35, 35') ein Stift oder ein Winkelsektor in Form eines Scheibenabschnitts ist und wobei der erste Winkelanschlag (29, 29') und der zweite Winkelanschlag (31, 31') die winkeligen Enden eines Winkelsektors in Form eines Scheibenabschnitts bilden.

3. Aktivierungswerkzeug (3, 3') nach dem vorhergehenden Anspruch, wobei sich die Winkelaufnahme (33, 33') über einen Winkel zwischen 90° und 300° um die Drehachse (XX) erstreckt.

4. Aktivierungswerkzeug (3, 3') nach einem der vorhergehenden Ansprüche, wobei das Antriebselement (7) einen Elektromotor (21) aufweist.

5. Aktivierungswerkzeug (3, 3') nach einem der vorhergehenden Ansprüche, wobei die Erfassungsmittel (19) einen ersten Sensor (51) für die Winkelposition der ersten Welle (9) und einen zweiten Sensor (53) für die Winkelposition der zweiten Welle (15) aufweisen.

6. Aktivierungswerkzeug (3, 3') nach einem der vorhergehenden Ansprüche, wobei die Erfassungsmittel (19) einen Drehmomentsensor (37) aufweisen, der so konfiguriert ist, dass er das vom Elektromotor (21) ausgeübte Drehmoment misst, und/oder einen Stromsensor (39) aufweisen, der so konfiguriert ist, dass er den vom Elektromotor (21) verbrauchten Strom misst.

7. Aktivierungswerkzeug (3, 3') nach einem der vorhergehenden Ansprüche, wobei die Drehgeschwindigkeit des magnetischen Aktivierungselements (11) im ersten Modus weniger als oder gleich 30 Umdrehungen pro Minute, vorzugsweise weniger als oder gleich 18 Umdrehungen pro Minute, beträgt.

8. Aktivierungswerkzeug (3, 3') nach einem der vorhergehenden Ansprüche, das ein Kugellager (16) aufweist, das auf der zweiten Welle (15) angeordnet ist.

9. Distraktionsanordnung (1, 1') mit:
- einem Aktivierungswerkzeug (3, 3') nach einem der vorhergehenden Ansprüche, wobei das magnetische Aktivierungselement (11) ein Permanentmagnet ist, der in einer Richtung (Da) senkrecht zur Drehachse (XX) magnetisiert wird, und
- einen Platten-Distraktor (5), der eine erste Befestigungsplatte (41), eine zweite Befestigungsplatte (43), ein Distraktionselement (45), das so konfiguriert ist, dass es den Abstand zwischen der ersten Befestigungsplatte (41) und der zweiten Befestigungsplatte (43) ändert, aufweist, und ein entferntes magnetisches Element (23) in Form eines Permanentmagneten, das so konfiguriert ist, dass es das Distraktionselement (45) durch Drehung um sich selbst um eine Distraktionsachse (YY) antreibt, wobei das entfernte magnetische Element (23) ein Permanentmagnet ist, der in einer Richtung (Dd) senkrecht zu der Distraktionsachse (YY) magnetisiert ist.

## Claims

1. Activation tool (3, 3') for a bone expansion apparatus, preferably for a plate distractor (5), the activation tool (3, 3') comprising:
- a drive member (7) provided with a drive output,
- a first shaft (9) configured to be driven in rotation about a rotation axis via the drive output,
- an activation magnetic element (11) configured to interact, preferably coaxially, with a remote magnetic element (23) supported by the bone expansion apparatus,
- a second shaft (15), arranged coaxially to the first shaft (9), the second shaft (15) supporting the activation magnetic element (11),
**characterised in that** the activation tool (3, 3') comprises:
- a coupling device (17) interposed between the first shaft (9) and the second shaft (15), which is configured to couple the first shaft (9) and the second shaft (15) in a first mode, referred to as the activation mode, when the first shaft (9) and the second shaft (15) exhibit the same rotational speed, and is configured to angularly decouple the first shaft (9) and the second shaft (15) in a second mode, referred to as the failure mode, when a rotational speed of the second shaft (15) is higher than the rotational speed of the first shaft (9), and
- means (19) for detecting the switch from the first mode to the second mode,
the coupling device (17) comprising:
- a first coupling member (25, 25') supported by the first shaft (9), and
- a second coupling member (27, 27') supported by the second shaft (15),
one selected from the first coupling member (25, 25') and the second coupling member (27, 27') comprising a first angular stop (29, 29') and a second angular stop (31, 31'), the first angular stop (29, 29') and the second angular stop (31, 31') being angularly offset and delimiting between them an angular housing (33, 33') extending over an angle of at least 90° about the rotation axis ,
the other selected from the first coupling member (25, 25') and the second coupling member (27, 27') comprising a projecting element (35, 35'),
the projecting element (35, 35') being engaged in the angular housing (33, 33') such that the second shaft (15) pivots freely about the rotation axis (XX) between a first angular position in which the projecting element (35, 35') is in contact with the first angular stop (29, 29') and a second angular position in which the projecting element (35, 35') is in contact with the second angular stop (31, 31').

2. Activation tool (3, 3') according to the preceding claim, wherein the projecting element (35, 35') is a pin or an angular sector consisting of a disc portion, and wherein the first angular stop (29, 29') and the second angular stop (31, 31') form the angular extremities of an angular sector consisting of a disc portion.

3. Activation tool (3, 3') according to the preceding claim, wherein the angular housing (33, 33') extends over an angle of between 90° and 300° about the rotation axis (XX).

4. Activation tool (3, 3') according to any one of the preceding claims, wherein the drive member (7) comprises an electric motor (21).

5. Activation tool (3, 3') according to any one of the preceding claims, wherein the detection means (19) comprise a first sensor (51) of the angular position of the first shaft (9) and a second sensor (53) of the angular position of the second shaft (15).

6. Activation tool (3, 3') according to any one of the preceding claims, wherein the detection means (19) comprise a torque sensor (37) configured to measure the torque exerted by the electric motor (21) and/or a current sensor (39) configured to measure the current consumed by the electric motor (21).

7. Activation tool (3, 3') according to any one of the preceding claims, wherein the rotational speed of the activation magnetic element (11) in the first mode is less than or equal to 30 revolutions per minute, preferably less than or equal to 18 revolutions per minute.

8. Activation tool (3, 3') according to any one of the preceding claims, which comprises a ball bearing (16) arranged on the second shaft (15).

9. Distraction assembly (1, 1') comprising:
- an activation tool (3, 3') according to any one of the preceding claims, wherein the activation magnetic element (11) is a permanent magnet magnetised along a direction (Da) perpendicular to the rotation axis (XX), and
- a plate distractor (5), which comprises a first attachment plate (41), a second attachment plate (43), a distraction member (45) configured to modify the distance between the first attachment plate (41) and the second attachment plate (43), and a remote magnetic element (23) consisting of a permanent magnet, configured to drive the distraction member (45) in rotation about itself about a distraction axis (YY), the remote magnetic element (23) being a permanent magnet magnetised along a direction (Dd) perpendicular to the distraction axis (YY).
